(19) Europäisches Patentamt
European Patent Office
Office européen des brevets

(11) Numéro de publication: **0 516 506 B1**

(12) **FASCICULE DE BREVET EUROPEEN**

(45) Date de publication du fascicule du brevet: **13.09.95**

(51) Int. Cl.[6]: **C07C 15/00**

(21) Numéro de dépôt: **92401372.5**

(22) Date de dépôt: **20.05.92**

# (54) Procédé catalytique de production d'hydrocarbures liquides à partir du gaz naturel.

(30) Priorité: **21.05.91 FR 9106192**
**21.05.91 FR 9106193**

(43) Date de publication de la demande:
**02.12.92 Bulletin 92/49**

(45) Mention de la délivrance du brevet:
**13.09.95 Bulletin 95/37**

(84) Etats contractants désignés:
**DE DK GB IT NL**

(56) Documents cités:
**EP-A- 0 351 312**
**EP-A- 0 357 488**
**WO-A-86/05176**
**FR-A- 2 629 451**

(73) Titulaire: **INSTITUT FRANCAIS DU PETROLE**
**4, Avenue de Bois Préau**
**F-92502 Rueil-Malmaison (FR)**

(72) Inventeur: **Alario, Fabio**
**70 bis, rue Georges Clémenceau**
**F-94210 La Vareene (FR)**
Inventeur: **Cameron, Charles**
**118, rue d'Assas**
**F-75006 Paris (FR)**
Inventeur: **Martino, Germain**
**Batiment Condé,**
**80, avenue F. Lefebvre**
**F-78300 Poissy (FR)**

**EP 0 516 506 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de production d'hydrocarbures liquides à partir du gaz naturel. Plus spécifiquement cette invention concerne la transformation du gaz naturel, dont le constituant majeur est le méthane, en produits liquides plus facilement transportables. Encore plus spécifiquement, cette invention concerne un procédé caractérisé en ce que :

(a) on sépare le gaz naturel en, au moins deux fractions, une première fraction du gaz enrichie en méthane et une deuxième fraction enrichie en alcanes $C_2^+$ (éthane, propane et alcanes supérieurs),

(b) on oxyde sélectivement au moins une partie du méthane par de l'oxygène moléculaire en présence d'un catalyseur de couplage oxydant du méthane,

(c) on mélange, au moins en partie, la fraction enrichie en alcanes $C_2^+$ à l'effluent de l'étape d'oxydation sélective quand au moins 80 % de l'oxygène moléculaire introduit à l'étape (b) ont déjà été consommés dans l'étape (b),

(d) on pyrolyse le mélange résultant de l'étape (c),

(e) après avoir amené la température du mélange de l'étape (d) à une température comprise entre 300 °C et 750 °C et plus particulièrement entre 450 °C et 600 °C, on transforme, en partie au moins, les oléfines et éventuellement en partie des alcanes $C_2^+$ , en aromatiques en présence d'un catalyseur d'aromatisation.

Les principes de base du procédé d'oxypyrolyse du gaz naturel, qui combine l'oxydation sélective du méthane et la pyrolyse des alcanes $C_2^+$ formés et ajoutés à l'effluent d'oxydation, sont décrits dans trois brevets français (2 629 451, 2.636.627 et 2.641.531) dans WO-A-86/05176 et dans un article (Appl. Catal., vol. 58 (1990) 269).

Dans une première étape, étape (a), de ce procédé, le gaz naturel est séparé en au moins deux fractions, la première étant du méthane appauvri en alcanes $C_2^+$ (éthane, propane et alcanes supérieurs) et la deuxième fraction étant composée des alcanes supérieurs $C_2^+$ appauvris en méthane. Une partie au moins de la première fraction est ensuite mélangée avec de l'oxygène (pur ou enrichi) jusqu'à 0,4 mol d'oxygène moléculaire par mole de carbone. Le mélange peut contenir avantageusement des quantités importantes de vapeur d'eau. La quantité molaire de vapeur d'eau par rapport au méthane peut être entre 0 et 4, de préférence entre 0 et 2, de préférence encore entre 0 et 1 et plus particulièrement entre 0,05 et 0,50. La vapeur d'eau permet la manipulation des mélanges oxygène/hydrocarbures avec un degré de sécurité plus important et permet l'augmentation simultanée de la sélectivité en produits de couplage et de la conversion du méthane dans l'étape d'oxydation sélective du méthane.

La deuxième étape du procédé, étape (b), consiste à consommer l'oxygène moléculaire par une réaction d'oxydation catalytique sélective à des températures au moins supérieures à 650 °C, de préférence supérieures à 750 °C et de préférence encore supérieures à 800 °C. Généralement la pression est entre 1 et 15 bars, préférentiellement entre 1 et 10 bars et plus particulièrement entre 1 et 4 bars. Cette réaction, généralement appelée couplage oxydant du méthane, est effectuée en présence d'un catalyseur stable à haute temperature. Les catalyseurs stables à haute température sont généralement ceux qui contiennent au moins un oxyde réfractaire tel que la magnésie, l'oxyde de calcium, l'oxyde de strontium et les autres oxydes trouvés dans le tableau 3 de l'article paru dans Appl. Catal., vol. 67 (1990) 47. Les catalyseurs d'un intérêt particulier pour le couplage oxydant du méthane sont, entre autres, ceux décrits dans le brevet français (2 629 451), et dans les articles parus dans Appl. Catal., vol. 67 (1990) 47 et Chem. Soc. Rev., vol. 18 (1989) 251.

L'étape d'oxydation sélective peut être effectuée dans un réacteur à lit fixe, un réacteur à lit mobile ou un réacteur à lit transporté. L'utilisation d'un réacteur à lit fixe est particulièrement avantageuse dans le cas où le catalyseur n'a pas de bonnes propriétés de résistance mécanique et pour des conversions par passe du méthane relativement faibles, par exemple inférieures à 20 %. Les réacteurs à lit mobile, tels que ceux à lit bouillonnant ou à lit transporté, sont très intéressants à mettre en oeuvre quand la conversion par passe est, par exemple, supérieure à 20 %. La circulation du catalyseur permet un meilleur contrôle de la température par échange thermique entre la charge, le catalyseur et les effluents.

Quel que soit le réacteur utilisé pour l'étape d'oxydation sélective du méthane, cette étape d'oxydation sélective est fortement exothermique. Donc, il est très important d'abaisser la température de l'effluent pour limiter la formation d'acétylène et de coke qui peuvent être formés aux temps de contact élevés à température élevée. Pour cette raison il est souvent avantageux d'injecter, dans cet effluent chaud, au moins une partie de la deuxième fraction, composée d'alcanes supérieurs $C_2^+$ appauvris en méthane, provenant de la première étape. Les hydrocarbures $C_2^+$ , généralement paraffiniques, servent à abaisser la température en effectuant une trempe thermochimique, autrement dit : ils absorbent la chaleur dégagée par l'étape d'oxydation et sont transformés en oléfines et en hydrogène.

La troisième étape, étape (c), consiste à additionner au moins en partie les paraffines $C_2^+$ obtenues à l'étape (a) dans l'effluent d'oxydation sélective du méthane quand au moins 80 %, et de préférence au moins 95 %, de l'oxygène moléculaire introduit à l'étape (b) ont déjà été consommés dans la deuxième étape. Il y a trois avantages pour ce mode opératoire :

(a) il n'est pas nécessaire d'opérer, dans l'étape (b) d'oxydation sélective du méthane, en présence des hydrocarbures $C_2^+$ plus oxydables que le méthane;

(b) l'addition des hydrocarbures $C_2^+$ de l'effluent d'oxydation sélective du methane, après qu'au moins 80 % et de préférence au moins 95 % de l'oxygène moléculaire introduit à l'étape (b)aient déjà été consommés, permet l'utilisation de la majorité de l'oxygène moléculaire pour l'activation du méthane et non pas pour la déshydrogénation des alcanes $C_2^+$ ;

(c) l'addition des hydrocarbures $C_2^+$ dans l'effluent de l'oxydation sélective du méthane, après qu'au moins 80 % et de préférence au moins 95 % de l'oxygène moléculaire introduit à l'étape (b) aient déjà été consommés, permet la transformation par absorption de chaleur des alcanes $C_2^+$ en oléfines et en hydrogène, ce dernier pouvant être utilisé pour la récupération de carbone, par exemple en hydrogénant le CO en méthane.

La quatrième étape. étape (d), consiste à maintenir le temps de séjour des effluents combinés pendant un temps suffisamment long pour permettre de préférence l'obtention par pyrolyse d'un rapport molaire éthylène/ éthane d'au moins 1,2 et de préférence supérieur à 1,4. Au cours de cette étape, le temps de séjour est compris généralement entre 50 millisecondes et 10 secondes, de préférence entre 100 millisecondes et 2 secondes. entre 50 millisecondes et 10 secondes, de préférence entre 100 millisecondes et 2 secondes.

Selon une méthode de production d'hydrocarbures liquides à partir du gaz naturel, qui ne fait pas partie de cette invention, les effluents gazeux sortant de la quatrième étape, étape (d), sont amenés à une température inférieure à 100 ° C, comprimés et introduits dans le système de séparation. Le gaz comprimé est ensuite débarrassé de l'eau et du $CO_2$ avant la réalisation d'une séparation des hydrocarbures supérieurs ($C_2$, $C_2=$, $C_3$, $C_3=$ et hydrocarbures supérieurs) et des gaz légers (CO, $H_2$, $CH_4$). Les hydrocarbures supérieurs sont ensuite :

1. séparés pour produire des oléfines, (éthylène, propylène et oléfines supérieures) et des alcanes recyclables,
2. oligomérisés et/ou aromatisés pour l'obtention des hydrocarbures liquides et des alcanes recyclables, ou
3. dimérisés pour produire des oléfines $C_4^+$ liquides et des alcanes recyclables.

Selon la méthode de la présente invention, l'effluent provenant de l'oxypyrolyse, à l'état gazeux et contenant des gaz parmi lesquels des hydrocarbures tels que l'éthylène, l'éthane, le propane et le propylène, est avantageusement traité sur un catalyseur d'aromatisation. Il est en effet d'un grand intérêt de valoriser ces hydrocarbures $C_2$-$C_3$ en produits liquides, grands intermédiaires pétrochimiques par exemple.

Lesdits effluents de l'étape (d), qui sont ensuite utilisés pour la charge de l'étape (e), sont constitués :

1. d'au moins 40 % et au plus 95 % en poids de l'ensemble méthane plus eau,
2. d'au moins 5 %, de préférence au moins 10 %, et de préférence encore au moins 15 % en poids de composés hydrocarbures non-paraffiniques,
3. moins de 1 %, de préférence moins de 0,5 %, et de préférence encore moins de 0,1 % en poids d'oxygène moléculaire, et
4. d'autres composés (en quantités variables dépendant de la charge initiale de l'étape (a) et des conditions de fonctionnement des étapes (a) à (d)), tels que : $N_2$, CO, $CO_2$, $H_2$ et les alcanes $C_2^+$ .

La cinquième étape, étape (e), consiste, donc, à amener les effluents de l'étape (d) à une température comprise entre 300 ° C et 750 ° C, et plus particulièrement entre 450 ° C et 600 ° C, puis l'effluent est mis en contact avec un catalyseur d'aromatisation qui permet la transformation, en partie au moins, des oléfines en hydrocarbures liquides. La transformation de ces oléfines et éventuellement, en partie au moins, des alcanes $C_2^+$ en hydrocarbures liquides peut avoir des conséquences importantes sur les dimensions du système de séparation. De plus, le groupement d'une unité chaude, telle que l'unité d'aromatisation, avec d'autres unités chaudes avant les étapes froides de séparation, a des conséquences positives sur l'investissement nécessaire pour l'ensemble opérationnel de valorisation du gaz naturel.

L'invention possède deux avantages importants. La transformation au moins en partie des oléfines et éventuellement des alcanes $C_2^+$ en aromatiques avant d'entrer dans le système de séparation permet :

1. la réduction des dimensions du système de séparation,
2. la réduction des utilités demandées grâce au regroupement d'une unité chaude (aromatisation) avec d'autres unités chaudes (oxydation, pyrolyse).

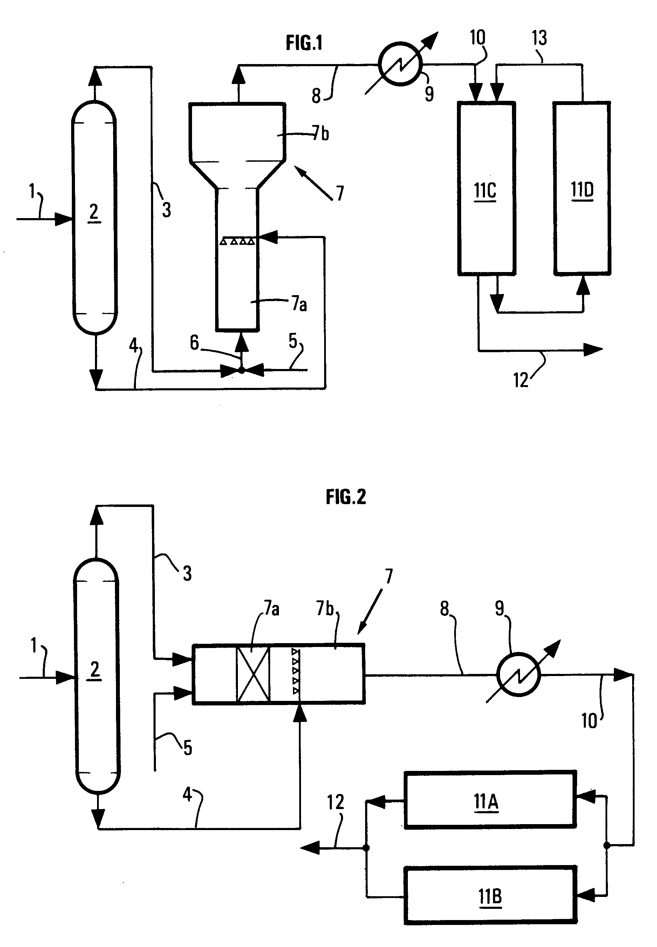

Les exemples suivants non limitatifs illustrent différents modes de réalisation qui peuvent être employés pour obtenir des hydrocarbures liquides à partir du gaz naturel selon l'invention. Ces exemples sont illustrés par les figures 1 et 2.

Une mise en oeuvre préférée de la présente invention consiste à utiliser un réacteur à lit bouillonnant pour les étapes d'oxydation sélective et de pyrolyse (oxypyrolyse) et un réacteur de type lit mobile pour l'aromatisation. Dans cette mise en oeuvre, le gaz enrichi en méthane (3) provenant de la séparation du gaz naturel (1) dans le séparateur (2) est mélangé à de l'oxygène moléculaire (5). Dans le cas où il est avantageux d'ajouter la vapeur d'eau dans la charge, celle-ci est généralement ajoutée au méthane ou à l'oxygène, ou au méthane et à l'oxygène avant de mélanger le méthane à l'oxygène. Il est très important de contrôler la température du mélange gazeux avant qu'il entre dans le réacteur. Pour ceci, on peut chauffer indépendamment les gaz contenant le méthane (3) et l'oxygène (5) ou l'ensemble des gaz (6). Les gaz préchauffés à une température généralement inférieure à 750 °C et de préférence inférieure à 600 °C, sont mis en contact avec le catalyseur de couplage oxydant du méthane dans la partie inférieure (7a) du réacteur (7) sur la figure 1 ou dans la partie en amont (7a) du réacteur (7) sur la figure 2.

Le catalyseur pour ce type de mise en oeuvre doit avoir une bonne résistance mécanique. La taille des particules est généralement entre 20 microns et 4 mm de diamètre. La taille des particules de catalyseur est variable en fonction des conditions opératoires de l'unité et en fonction de la densité du catalyseur.

Bien qu'on ne puisse pas mentionner tous les catalyseurs du couplage oxydant du méthane potentiellement intéressants pour une application dans un réacteur à lit bouillonnant, on peut citer les catalyseurs décrits dans le brevet français (2 629 451), dans Appl. Catal., vol. 76 (1990) 47, dans Chem. Soc. Rev., vol. 18 (1989) 251, et par exemples des catalyseurs tels que : $BaCO_3/Al_2O_3$ (généralement, mais pas obligatoirement, en présence de quelques ppm d'une source de chlore dans la charge) ; $Pb/Al_2O_3$ (souvent en présence d'un métal alcalin et/ou un anion contenant du soufre ou du phosphore) ; des oxydes mixtes contenant Na, B, Mg et Mn (tels que $NaB_2Mg_4Mn_2O_x$); La/MgO (souvent en présence d'un ou plusieurs oxydes des métaux alcalino-terreux et/ou alcalins et/ou d'autres oxydes de lanthanides, et éventuellement le bore) ; les combiniaisons Na ou K, un ou plusieurs oxydes des métaux alcalino-terreux, et éventuellement le bore; des perovskites $MCeO_3$ (ou M = Sr ou Ba) et les oxydes mixtes contenant du thorium ou du yttrium.

Après avoir subi l'oxydation sélective dans le réacteur (7) et à un endroit où au moins 80 % de l'oxygène moléculaire introduit à l'étape (b) ont été consommés, une partie au moins de la fraction du gaz enrichie en alcanes $C_2^+$ (4) est additionnée dans le réacteur. L'ajout de cette fraction peut être effectué dans le lit expansé du catalyseur (figure 1) ou à un niveau supérieur au catalyseur (telle que dans la zone de désengagement).

Après un temps de résidence suffisamment long, dans le réacteur (étape (d) du procédé), pour obtenir le taux d'oléfines désiré, les gaz sont conduits par la ligne (8) à un échangeur (9) où la température de ces gaz est abaissée à une température comprise entre 300 °C et 750 °C, et plus particulièrement entre 450 °C et 600 °C. Lesdits effluents sont ensuite envoyés sur la figure 1 par la ligne (10) vers le réacteur d'aromatisation (11c). Si la pression de fonctionnement du réacteur d'aromatisation (11c) est supérieure à la pression dans la ligne (10), lesdits effluents peuvent être avantageusement comprimés à ce moment.

Dans la figure 1, le réacteur d'aromatisation (11c), dans une des mises en oeuvre préférées, reçoit la charge à aromatiser avec du catalyseur frais par la partie supérieure du réacteur. De cette manière, le catalyseur désactivé par col:age est enlevé en bas du réacteur. Le catalyseur désactivé subit ensuite une régénération en (11d) avant d'être chargé à nouveau par la ligne (13) dans la partie supérieure du réacteur (11c). La charge, entrant dans le réacteur, est mise en contact avec le catalyseur d'aromatisation.

Bien que tous les catalyseurs connus d'aromatisation conviennent pour la présente invention, on préfère les catalyseurs décrits dans les demandes de brevets français 2.676.746, 2.676.747 et 2.676.748 de la demanderesse. Le catalyseur d'aromatisation est mis en oeuvre pour la réaction d'aromatisation des gaz d'oxypyrolyse. Cette réaction revêt un intérêt particulier car elle permet de valoriser des hydrocarbures gazeux en des produits liquides à plus haute valeur ajoutée (benzène, toluène, xylènes principalement).

La charge, effluent d'oxypyrolyse et contenant, parmi d'autres composés, de l'éthylène et/ou de l'éthane et/ou du propylène, est mise en contact avec le catalyseur d'aromatisation à une température comprise entre 300 °C et 750 °C, et plus particulièrement entre 450 °C et 600 °C, et avec un débit massique horaire de charge par rapport au poids de catalyseur (PPH) compris entre 0,5 et 150 $h^{-1}$, de préférence entre 2 et 80 $h^{-1}$. La pression de fonctionnement sera avantageusement comprise entre 1 et 18 bars, et de préférence entre 1 et 12 bars.

L'effluent dudit réacteur d'aromatisation (11c), enrichi en produits aromatiques, est transféré par le conduit (12) vers un système de séparation.

Une autre mise en oeuvre préférée de la présente invention consiste (figure 2) à utiliser un réacteur à lit fixe (7) pour les étapes d'oxydation sélective et de pyrolyse, de préférence mais pas nécessairement dans

la même enceinte (donc ici 2 zones distinctes 7a et 7b). Selon une technique particulière (cf figure 2), l'aromatisation peut être effectuée dans au moins un réacteur décrit par (11a) et (11b), pouvant être mis "hors circuit" pour régénérer le catalyseur qu'il contient ("swing reactor" en langue anglaise). Dans cette mise en oeuvre de la présente invention, les catalyseurs utilisés pour l'oxydation sélective du méthane et l'aromatisation en partie au moins des oléfines, et éventuellement en partie des alcanes $C_2^+$ , ne doivent pas nécessairement être très résistants à l'attrition. Il convient, donc, que les catalyseurs soient utilisés sous forme d'extrudés, de concassés ou de grains. Tous les catalyseurs pour une application dans un réacteur à lit bouillonnant peuvent être utilisés pour une application dans un réacteur à lit fixe. Le catalyseur pour ce type de mise en oeuvre ne nécessite pas une résistance mécanique particulièrement élevée.

Dans le cas où il est avantageux d'ajouter la vapeur d'eau dans la charge, celle-ci est généralement ajoutée au méthane ou à l'oxygène, ou au méthane et à l'oxygène avant de mélanger le méthane à l'oxygène. Il est très important de contrôler la température du mélange gazeux avant qu'il entre dans le réacteur. Pour ceci, dans le cas d'un réacteur à lit fixe, on chauffe indépendamment les gaz contenant le méthane (3) et l'oxygène (5) avant que le méthane soit mélangé à l'oxygène. Le mélange entre le méthane l'oxygène est effectué dans l'enceinte du réacteur (7) avant contact avec le catalyseur. Le mélange étant effectué dans le réacteur, le conduit (6) n'est éventuellement pas nécessaire pour cette mise en oeuvre. Néanmoins, il est souvent avantageux qu'une partie du préchauffage des gaz soit effectuée dans le réacteur. L'eau présente dans la charge est capable de recevoir, par rayonnement thermique, des calories dégagées par le catalyseur. Donc, par cette méthode une partie du préchauffage peut être effectuée dans le réacteur d'oxydation. Les gaz, préchauffés à une température généralement inférieure à 750 °C et de préférence inférieure à 600 °C, sont ensuite mis en contact avec le catalyseur du couplage oxydant du méthane dans le réacteur (7).

Après avoir subi l'oxydation sélective dans le réacteur (7) et à un endroit où au moins 80 % de l'oxygène moléculaire introduit à l'étape (b) ont été consommés, au moins une partie de la fraction du gaz enrichie en alcanes $C_2^+$ (4) est ajoutée dans le réacteur. L'endroit d'ajout de cette fraction se situe généralement après le lit catalytique pour raison de simplicité d'opération.

Après un temps de résidence suffisamment long pour obtenir le taux d'oléfines désiré (rapport molaire éthylène/éthane d'au moins 1,2), le gaz est acheminé vers le réacteur d'aromatisation par les lignes (8) et (10) au travers de l'unité (9). L'ensemble du réacteur d'aromatisation, décrit par (11a) et (11b), fonctionne en discontinu. La partie (11a) de l'ensemble du réacteur, par exemple, reçoit le gaz à aromatiser à une température et pendant un temps suffisamment long pour transformer une partie au moins des oléfines, et éventuellement une partie des alcanes $C_2^+$ , en aromatiques. Pendant une période, au moins, de cette transformation dans la partie (11a) de l'ensemble de réacteur, la partie (11b) est mise en régénération pour enlever le carbone qui s'est déposé sur le catalyseur durant le cycle précédent. Après cette étape de régénération, la partie (11b) est remise en service et ainsi de suite.

L'effluent aromatisé dans le réacteur d'aromatisation (11a et 11b) est ensuite envoyé vers le système de séparation par la ligne (12).

La charge, entrant dans le réacteur, est mise en contact avec un catalyseur d'aromatisation qui, dans la présente invention, est de préférence de type galloaluminosilicate et caractérisé par la composition suivante exprimée en poids :

(a) 0,01 à 10 % de gallium, de préférence 0,03 à 7 %,

(b) 0,1 à 99,49 % d'une matrice choisie dans le groupe formé par l'alumine, la silice, la magnésie, une argile et toute combinaison d'au moins deux des composés précités et,

(c) 0,5 à 99,89 % d'une zéolithe de structure MFI, synthétisée en milieu $OH^\ominus$ ou $F^\ominus$ en absence ou en présence de composés organiques, dont la formule chimique approchée est généralement la suivante :

$$M^+_{(x+y)}(Si_{(96-(x+y))}Al_xGa_yO_{192})^{(x+y)-},$$

où M représente un cation alcalin et/ou un cation ammonium et/ou un proton, x est un nombre compris entre 0,1 et 12, y est un nombre compris entre 0 et 13,7.

La zéolithe MFI possède de préférence une teneur en fluor comprise entre 0,01 et 2 % en poids, de préférence 0,02 à 1,5 % en poids, le fluor étant incorporé de préférence lors de la synthèse.

La zéolithe MFI utilisée dans la présente invention peut être obtenue selon toutes les méthodes de préparation connues. Elle peut être par exemple synthétisée en milieu $OH^\ominus$ ou $F^\ominus$ en présence ou en absence de composés organiques.

La zéolithe ainsi obtenue peut être soumise telle quelle à un dépôt de gallium puis mise en forme par toutes les techniques connues de l'homme du métier. Cette mise en forme peut également intervenir avant

que le dépôt de gallium ait été effectué. Dans ce cas, on procède au dépôt de gallium sur le solide mis en forme.

La zéolithe MFI peut en particulier être mélangée à une matrice généralement amorphe, par exemple à une poudre humide de gel d'alumine. Le mélange est ensuite mis en forme, par exemple par extrusion au travers d'une filière. La teneur en zéolithe du support ainsi obtenue est généralement comprise entre environ 0,5 et 99,89 % et avantageusement comprise entre environ 40 et 90 % poids. La teneur en matrice du catalyseur est comprise entre 0,1 et 99,49 % et avantageusement entre environ 10 et 60 %. La mise en forme peut être réalisée avec d'autres matrices que l'alumine, telles que, par exemple la magnésie, la silice alumine, les argiles naturelles (kaolin, bentonite), et par des techniques telles que par exemple l'extrusion, le pastillage, la dragéification, la coagulation en gouttes (oil drop) ou le séchage par atomisation.

Le dépôt de gallium, sur la zéolithe MFI avant mise en forme ou bien sur le support après mise en forme, est effectué par tout procédé connu de l'homme du métier et permettant le dépôt du métal dans la zéolithe. On peut utiliser la technique d'échange cationique avec compétition où l'agent compétiteur est de préférence le nitrate d'ammonium, ou encore les techniques de dépôt de gallium sur le catalyseur par imprégnation ou par précipitation. Les solutions d'échange ou d'imprégnation du gallium peuvent être préparées à partir de composés du gallium tels que par exemple l'oxyde de gallium, le nitrate de gallium, le sulfate de gallium, des halogénures de gallium ou l'hydroxyde de gallium. Ces techniques d'échange ionique ou d'imprégnation ou de précipitation peuvent également être utilisées pour déposer le métal directement sur la poudre de zéolithe, avant son mélange éventuel avec une matrice. La teneur en gallium déposée sur le catalyseur à l'issue de ou des étapes d'échange ionique et/ou d'imprégnation ou de précipitation se situe entre 0,01 et 10 % en poids par rapport à l'ensemble du catalyseur, et de préférence entre 0,03 % et 7,0 % en poids.

Le catalyseur, obtenu par les procédures précédentes et pouvant éventuellement subir un traitement de calcination sous air à une température généralement comprise entre 350 °C et 690 °C, est mis en oeuvre pour la réaction d'aromatisation des gaz d'oxypyrolyse. Cette réaction revêt un intérêt particulier car elle permet de valoriser des hydrocarbures gazeux en des produits liquides à plus haute valeur ajoutée (benzène, toluène, xylènes principalement).

Les autres exemples qui suivent précisent le procédé sans toutefois en limiter la portée.

Le catalyseur utilisé dans les exemples suivants renferme 20 % en poids d'une matrice et 80 % en poids de zéolithe, sous forme d'extrudés.

EXEMPLE 1 : Préparation du catalyseur d'aromatisation

On utilise une zéolithe MFI forme hydrogène fournie par la société Conteka sous la référence CBV1502. Cette zéolithe MFI est caractérisée par un rapport molaire Si/Al égal à 75, une teneur en sodium égale à 0,016% en poids et un volume poreux mesuré par adsorption d'azote à 77 K de 0,174 $cm^3g^{-1}$.

Cette zéolithe MFI est mélangée à une poudre humide de gel d'alumine. Le mélange est ensuite mis en forme par extrusion au travers d'une filière.

Après séchage et calcination du solide extrudé obtenu, le gallium est déposé sur cette zéolithe par échange ionique. La solution d'échange est préparée à partir de nitrate de gallium 0,2 M. Le pH de la solution est ajusté à 2,2 avec de l'ammoniaque.

La teneur en gallium, atteinte après quatre échanges, dans le catalyseur A ainsi obtenu était de 3,2% en poids.

EXEMPLE 2 : Transformation d'un effluent d'oxypyrolyse

Le catalyseur de l'exemple 1 a été mis en oeuvre pour l'aromatisation d'un effluent d'oxypyrolyse (10) , dont la composition pondérale est indiquée dans le tableau 1. Dans ce qui suit, l'effluent d'oxypyrolyse est dénommé "charge", et ses produits de transformation sur le catalyseur d'aromatisation sont dénommés "produits".

TABLEAU 1

| | Charge % en poids |
|---|---|
| $C_1$ | 62,4 |
| $C_2^=$ | 13,4 |
| $C_2$ | 5,06 |
| $C_3^=$ | 0,82 |
| $H_2$ | 1,08 |
| $O_2$ | 0,04 |
| CO | 0,90 |
| $CO_2$ | 4,70 |
| $H_2O$ | 11,6 |

Le catalyseur d'aromatisation a été chargé dans un réacteur à lit fixe en acier inoxydable (11a) et les conditions opératoires étaient les suivantes :

- Température : 530 ° C,
- Pression : atmosphérique,
- Débit horaire de charge liquide égal à 20 fois le poids du catalyseur.

Les résultats, en termes de pourcentages massiques des produits trouvés dans la ligne (12), sont présentés dans le tableau 2.

TABLEAU 2

| | Produits % en poids |
|---|---|
| $C_1$ | 63,6 |
| $C_2^=$ | 1,92 |
| $C_2$ | 5,64 |
| $C_3^=$ | 0,73 |
| $C_3$ | 1,04 |
| $C_4^=$ | 0,50 |
| $C_4$ | 0,62 |
| $C_{5+}$ non aromatiques | 0,31 |
| Aromatiques | 7,19 |
| $H_2$ | 1,21 |
| CO | 0,81 |
| $CO_2$ | 4,83 |
| $H_2O$ | 11,6 |

**Revendications**

**1.** Procédé de production d'hydrocarbures liquides à partir du gaz naturel caractérisé en ce que :
(a) on sépare le gaz naturel en, au moins deux fractions, une première fraction du gaz enrichie en méthane et une deuxième fraction enrichie en alcanes $C_2^+$ (éthane, propane et alcanes supérieurs),
(b) on oxyde sélectivement au moins une partie du méthane par de l'oxygène moléculaire en présence d'un catalyseur de couplage oxydant du méthane,
(c) on mélange, au moins en partie, la fraction enrichie en alcanes $C_2^+$ à l'effluent de l'étape d'oxydation sélective quand au moins 80 % de l'oxygène moléculaire introduit à l'étape (b) ont déjà été consommés dans l'étape(b),
(d) on pyrolyse le mélange résultant de l'étape (c),
(e) après avoir amené la température du mélange de l'étape (d) à une température comprise entre 300 °C et 750 °C, on transforme, en partie au moins, les oléfines, et éventuellement en partie des alcanes $C_2^+$ , en aromatiques en présence d'un catalyseur d'aromatisation.

**2.** Procédé selon la revendication 1 dans lequel, au cours de l'étape (e), la température du mélange de l'étape (d) est amené à une température comprise entre 450 °C et 600 °C.

**3.** Procédé selon l'une des revendications 1 et 2 dans lequel, à l'issue de l'étape (a), une partie au moins de la première fraction (fraction méthane) est mélangée avec de l'oxygène dont la teneur peut atteindre 0,4 mol d'oxygène moléculaire par mole de carbone.

4. Procédé selon la revendication 3 dans lequel le mélange renferme une quantité molaire de vapeur d'eau comprise entre 0 et 4 par rapport au méthane.

5. Procédé selon l'une des revendications 1 à 4 dans lequel au moins une partie de la deuxième fraction ($C_2^+$) obtenue à l'étape (a) est injectée dans la zone d'oxydation sélective du méthane (étape (b)).

6. Procédé selon l'une des revendications 1 à 5 dans lequel, au cours de l'étape (d), on maintient le temps de séjour des effluents combinés obtenus à l'étape (c) jusqu'à l'obtention par pyrolyse d'un rapport molaire éthylène/éthane d'au moins 1.2, ledit temps de séjour étant compris entre 50 millisecondes et 10 secondes.

7. Procédé selon l'une des revendications 1 à 6 pour la production d'hydrocarbures liquides à partir du gaz naturel caractérisé en ce que :
   (a) on sépare le gaz naturel en, au moins deux fractions, une première fraction du gaz enrichie en méthane et une deuxième fraction enrichie en alcanes $C_2^+$ (éthane, propane et alcanes supérieurs),
   (b) on oxyde sélectivement au moins une partie du méthane par de l'oxygène moléculaire en présence d'un catalyseur de couplage oxydant du méthane,
   (c) on mélange, au moins en partie, la fraction enrichie en alcanes $C_2^+$ à l'effluent de l'étape d'oxydation sélective quand au moins 80 % de l'oxygène moléculaire introduit à l'étape (b) ont déjà été consommés dans l'étape (b),
   (d) on pyrolyse le mélange résultant de l'étape (c),
   (e) après avoir amené la température du mélange de l'étape (d) à une température comprise entre 300° C et 750° C, on transforme, en partie au moins, les oléfines, et éventuellement en partie des alcanes $C_2^+$, en aromatiques en présence d'un catalyseur d'aromatisation, qui contient :
      (•) 0,01 à 10 % en poids de gallium, de préférence 0,03 à 7 % en poids,
      (•) 0,1 à 99,49 % en poids d'une matrice choisie dans le groupe formé par l'alumine, la silice, la magnésie, une argile et toute combinaison d'au moins deux des composés précités et,
      (•) 0,5 à 99,89 % en poids d'une zéolithe de structure MFI, synthétisée en milieu $OH^{\ominus}$ ou $F^{\ominus}$ en absence ou en présence de composés organiques, dont la formule chimique approchée est généralement la suivante : $M^{+}_{(x+y)}(Si_{(96-(x+y))}Al_xGa_yO_{192})^{(x+y)-}$, où M représente un cation alcalin et/ou un cation ammonium et/ou un proton, x est un nombre compris entre 0,1 et 12, y est un nombre compris entre 0 et 13,7.

8. Procédé selon la revendication 7 dans lequel, au cours de l'étape (e), la température du mélange de l'étape (d), est amenée à une température comprise entre 480° C et 600° C.

9. Procédé selon l'une des revendications 7 et 8 dans lequel, au cours de l'étape (d), on maintient le temps de séjour des effluents combinés obtenus à l'étape (c), jusqu'à l'obtention par pyrolyse d'un rapport molaire éthylène/éthane d'au moins 1,2, ledit temps de séjour compris entre 50 millisecondes et 10 secondes.

10. Procédé selon l'une des revendications 7 à 9 dans lequel la zéolithe MFI utilisée à l'étape (e) a une teneur en fluor comprise entre 0,01 et 2 % en poids, le fluor étant incorporé au cours de la synthèse de ladite zéolithe.

## Claims

1. A method of producing liquid hydrocarbons from natural gas, characterised in that :
   (a) natural gas is separated into at least two fractions, a first fraction of gas enriched with methane and a second fraction enriched with C2+ alkanes (ethane, propane and higher alkanes)
   (b) at least part of the methane is oxidised selectively by molecular oxygen in the presence of a catalyst for oxidising linkage of methane
   (c) at least part of the fraction enriched with C2+ alkanes is mixed with the effluent from the selective oxidation stage, when at least 80% of the molecular oxygen introduced at stage (b) has been consumed at stage (b)
   (d) the mixture resulting from stage (c) is pyrolysed
   (e) when the temperature of the mixture from stage (d) has been brought to 300 to 750°C, at least part of the olefins and possibly part of the C2+ alkanes are converted to aromatics in the presence

of an aromatising catalyst.

2. The method of claim 1 wherein, during stage (e), the temperature of the mixture from stage (d) is brought to a temperature from 450 to 600 °C.

3. The method of claim 1 or 2 wherein, at the end of stage (a), at least part of the first fraction (the methane fraction) is mixed with oxygen, the content of which may be up to 0.4 mol of molecular oxygen per mol of carbon.

4. The method of claim 3, wherein the mixture contains a molar quantity of water vapour of from 0 to 4 relative to the methane.

5. The method of any of claims 1 to 4, wherein at least part of the second fraction (C2+) obtained at stage (a) is injected into the zone for selective oxidation of methane (stage b).

6. The method of any of claims 1 to 5 wherein, during stage (d), the dwell time of the combined effluents obtained at stage (c) is maintained until an ethylene/ethane molar ratio of at least 1.2:1 is obtained by pyrolysis, the dwell time being from 50 milliseconds to 10 seconds.

7. The method of any of claims 1 to 6 for producing liquid hydrocarbons from natural gas, characterised in that:

(a) natural gas is separated into at least two fractions, a first fraction of gas enriched with methane and a second fraction enriched with C2+ alkanes (ethane, propane and higher alkanes)

(b) at least part of the methane is oxidised selectively by molecular oxygen in the presence of a catalyst for oxidising linkage of methane

(c) at least part of the fraction enriched with C2+ alkanes is mixed with the effluent from the selective oxidation stage, when at least 80% of the molecular oxygen introduced at stage (b) has been consumed at stage (b)

(d) the mixture resulting from stage (c) is pyrolysed

(e) when the temperature of the mixture from stage (d) has been brought to 300 to 750 °C, at least part of the olefins and possibly part of the C2+ alkanes are converted to aromatics in the presence of an aromatising catalyst containing:

(*) 0.01 to 10%, Preferably 0.03 to 7% of gallium

(*) 0.1 to 99.49% of a matrix selected from the group formed by alumina, silica, magnesia, a clay and any combination of at least two of the above compounds and

(*) 0.5 to 99.89% of an MFI zeolite synthesised in an $OH_{\ominus}$ or $F_{\ominus}$ medium in the absence or presence of organic compounds, its approximate chemical formula generally being the following: $M^{+}_{(x+y)} (Si_{(96-(x+y))}\ Al_x\ Ga_y\ O_{192})^{(x+y)-}$ where M represents an alkaline cation and/or an ammonium cation and/or a proton, $\underline{x}$ is a number from 0.1 to 12 and $\underline{y}$ is a number from 0 to 13.7.

8. The method of claim 7 wherein, during stage (e), the temperature of the mixture from stage (d) is brought to a temperature from 480 to 600 °C.

9. The method of claim 7 or 8 wherein, during stage (d), the dwell time of the combined effluents obtained at stage (c) is maintained, until an ethylene/ethane molar ratio of at least 1.2:1 is obtained by pyrolysis, the dwell time being from 50 milliseconds to 10 seconds.

10. The method of any of claims 7 to 9, wherein the MFI zeolite used at stage (e) has a fluorine content of 0.01 to 2% by weight, the fluorine being incorporated during the synthesis of the zeolite.

## Patentansprüche

1. Verfahren zur Herstellung von flüssigen Kohlenwasserstoffen aus Erdgas, dadurch gekennzeichnet, daß

(a) man das Erdgas in wenigstens zwei Fraktionen, eine erste mit Methan angereicherte Gasfraktion und eine zweite mit $C_2^+$-Alkanen (Ethan, Propan und höheren Alkanen) angereicherte Fraktion, trennt,

(b) man selektiv wenigstens einen Teil des Methans durch molekularen Sauerstoff in Anwesenheit eines Kopplungskatalysators, der Methan oxidiert, oxidiert,

(c) man wenigstens teilweise die mit $C_2^+$-Alkanen angereicherte Fraktion mit dem Abgang des Schrittes zur selektiven Oxidation mischt, wenn schon wenigstens 80 % des in den Schritt (b) eingeführten molekularen Sauerstoffs in dem Schritt (b) verbraucht worden sind,
(d) man die aus dem Schritt (c) resultierende Mischung pyrolysiert,
(e) man, nachdem die Temperatur der Mischung des Schrittes (d) auf eine Temperatur einschließlich zwischen 300 °C und 750 °C gebracht worden ist, wenigstens teilweise die Olefine und gegebenenfalls teilweise die $C_2^+$-Alkane in Aromaten in Anwesenheit eines Katalysators zur Aromatisierung umwandelt.

**2.** Verfahren nach Anspruch 1, bei welchem die Temperatur der Mischung des Schrittes (d) im Laufe des Schrittes (e) auf eine Temperatur einschließlich zwischen 450 °C und 600 °C gebracht wird.

**3.** Verfahren nach einem der Ansprüche 1 und 2, bei welchem am Ausgang des Schrittes (a) wenigstens ein Teil der ersten Fraktion (Methanfraktion) mit dem Sauerstoff gemischt wird, dessen Gehalt 0,4 Mol molekularen Sauerstoffs pro Mol Kohlenstoff erreichen kann.

**4.** Verfahren nach Anspruch 3, bei welchem die Mischung eine molare Menge an Wasserdampf einschließlich zwischen 0 und 4 in bezug auf Methan enthält.

**5.** Verfahren nach einem der Ansprüche 1 bis 4, bei welchem wenigstens ein Teil der aus dem Schritt (a) erhaltenen zweiten Fraktion ($C_2^+$) in die Zone zur selektiven Oxidation des Methans (Schritt (b)) injiziert wird.

**6.** Verfahren nach einem der Ansprüche 1 bis 5, bei welchem man im Laufe des Schrittes (d) die Verweilzeit der in dem Schritt (c) erhaltenen kombinierten Abgänge bis zum Erhalt eines Molverhältnisses Ethylen/Ethan von wenigstens 1,2 durch Pyrolyse aufrechterhält, wobei die Verweilzeit einschließlich zwischen 50 Millisekunden und 10 Sekunden beträgt.

**7.** Verfahren zur Herstellung von flüssigen Kohlenwasserstoffen aus Erdgas gemäß einem der Ansprüche 1 bis 6, dadurch gekennzeichnet, daß
(a) man das Erdgas in wenigstens zwei Fraktionen, eine erste mit Methan angereicherte Gasfraktion und eine zweite mit $C_2^+$-Alkanen (Ethan, Propan und höheren Alkanen) angereicherte Fraktion, trennt,
(b) man selektiv wenigstens einen Teil des Methans durch molekularen Sauerstoff in Anwesenheit eines Kopplungskatalysators, der Methan oxidiert, oxidiert,
(c) man wenigstens teilweise die mit $C_2^+$-Alkanen angereicherte Fraktion mit dem Abgang des Schrittes zur selektiven Oxidation mischt, wenn schon wenigstens 80 % des in den Schritt (b) eingeführten molekularen Sauerstoffs in dem Schritt (b) verbraucht worden sind,
(d) man die aus dem Schritt (c) resultierende Mischung pyrolysiert,
(e) man, nachdem die Temperatur der Mischung des Schrittes (d) auf eine Temperatur einschließlich zwischen 300 °C und 750 °C gebracht worden ist, wenigstens teilweise die Olefine und gegebenenfalls teilweise die $C_2^+$-Alkane in Aromaten in Anwesenheit eines Katalysators zur Aromatisierung umwandelt, der enthält:
(■) 0,01 bis 10 Gew.-% Gallium, vorzugsweise 0,03 bis 7 Gew.-%,
(■) 0,1 bis 99,49 Gew.-% einer Matrix, die aus der Gruppe ausgewählt ist, welche durch das Aluminiumoxid, das Siliciumdioxid, das Magnesiumoxid, einem Ton und jede Kombination wenigstens zwei der vorerwähnten Verbindungen gebildet ist, und
(■) 0,5 bis 99,89 Gew.-% eines Zeoliths der MFI-Struktur, der in $OH^-$- oder $F^-$-Milieu in Abwesenheit oder in Anwesenheit von organischen Verbindungen synthetisiert ist, dessen angenäherte chemische Formel im wesentlichen die folgende ist: $M^+_{(x+y)}(Si_{(96-(x+y))}Al_xGa_yO_{192})^{(x+y)-}$, wobei M ein Alkalikation und/oder ein Ammoniumkation und/oder ein Proton ist, x eine Zahl einschließlich zwischen 0,1 und 12 ist, y eine Zahl einschließlich zwischen 0 und 13,7 ist.

**8.** Verfahren nach Anspruch 7, bei welchem die Temperatur der Mischung des Schrittes (d) im Laufe des Schrittes (e) auf eine Temperatur einschließlich zwischen 480 °C und 600 °C gebracht wird.

**9.** Verfahren nach einem der Ansprüche 7 und 8, bei welchem man im Laufe des Schrittes (d) die Verweilzeit der aus dem Schritt (c) erhaltenen kombinierten Abgänge bis zum Erhalt eines Molverhältnisses Ethylen/Ethan von wenigstens 1,2 durch Pyrolyse aufrechterhält, wobei die Verweilzeit einschließlich zwischen 50 Millisekunden und 10 Sekunden beträgt.

**10.** Verfahren nach einem der Ansprüche 7 bis 9, bei welchem der in dem Schritt (e) verwendete MFI-Zeolith einen Gehalt an Fluor einschließlich zwischen 0,01 und 2 Gew.-% enthält, wobei das Fluor im Laufe der Synthese des Zeoliths eingebunden wird.

FIG.1

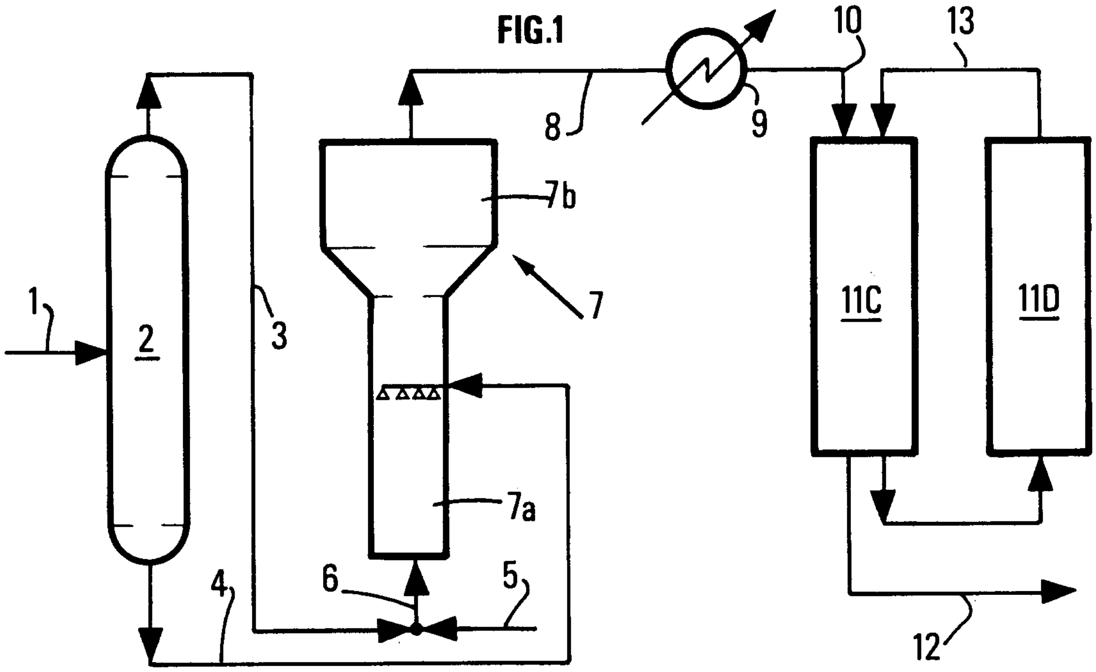


FIG.2